# EUROPEAN PATENT SPECIFICATION

(11) **EP 0 623 177 B1**
(45) Date of publication and mention of the grant of the patent: **08.10.1997**
(21) Application number: 93902411.3
(22) Date of filing: 18.01.1993
(51) Int. Cl.: C12Q 1/68

(54) **Reversibly bound in dessicated form to a solid support nucleid acid hydridisation probe, method using the same and kit therefor**
An einem festen Träger reversibel gebundene getrocknete Nucleinsäurehybridisierungsprobe, eine solche Probe verwendendes Verfahren und dafür geeigneter Kit
Sonde d'hybridation d'acide nucléique sous forme desséchée liée de manière réversible à un support solide, méthode la mettant en oeuvre et kit approprié

(30) Priority: 18.01.1992 GB 9201073
(43) Date of publication of application: 09.11.1994
(73) Proprietor: CYTOCELL LIMITED, Lewknor, Oxfordshire OX9 5TS (GB)
(72) Inventor: CARDY, Donald Leonard Nicholas, Aston-le-Walls, Northamptonshire N11 6UF (GB)
(74) Representative: Matthews, Heather Clare
(86) International application number: GB9300112
(87) International publication number: WO9314223

(56) References cited:
- EP-A- 0 111 340
- EP-A- 0 194 545
- EP-A- 0 298 669
- BIOLOGICAL ABSTRACTS, vol. 89, no. 5, 1990, Philadelphia, PA (US); no. 44713/

## Description

### Field of the Invention

The invention relates to methods of performing nucleic acid hybridisation assays, novel compositions for use in said assays, and kits for performing said assays.

### Background of the Invention

Nucleic acid hybridisation assays currently find many different applications, especially in the area of detection and diagnosis of infectious or genetic diseases.

The detection or diagnosis of genetic disease, involving the analysis of chromosomes in mammalian cells, usually requires the culturing of cells in nutrient medium in the presence of substances designed to block the cell cycle progression at mitosis, for example colchicine. At this stage, the chromosomes in mammalian cells are condensed and easily identifiable by staining. Without cell culture, a low frequency of cells in mitosis can be found although with varying difficulty depending on the cell type. Thus, it is routine in clinical cytogenetics laboratories for the analysis of peripheral blood cells or for the analysis of cells from amniocentesis samples (amniocytes) to culture these cell types for several weeks prior to chromosome analysis.

Standard methods of chromosome analysis require the deposition of fixed cells onto microscope slides and the viewing of mitotic cells by microscopy. Whilst chromosomes are readily visible in mitotic cells, the identification of individual chromosomes is more difficult and commonly requires matching pairs of chromosomes on the basis of size and G (Giemsa)-banding pattern. This is often done by photographing a stained chromosome spread and then manually pairing chromosomes from the photograph. Considerable skill is often required to identify chromosomal abnormalities in cells, for example in translocations of one section of a chromosome to another. In the 9:22 translocation associated with chronic myelogonous leukemia, for example, the cytogeneticist must identify the shortened chromosome 22 associated with a piece of chromosome 9 (the "Philadelphia chromosome"). Less extensive abnormalities within chromosomes, such as small deletions or mutations within genes, cannot be identified by standard visual methods for chromosomal analysis.

The use of hybridisation probes for chromosome analysis has greatly facilitated the identification of individual chromosomes in mitotic cells and the analysis of abnormalities such as translocations. For example, DNA probes have been developed which hybridise to centromeric regions of individual chromosomes (van Dekken and Bauman, Cytogenet. Cell Genet., volume 48 (1988) p188-189) or which hybridise to specific regions along the whole length of individual chromosomes (Pinkel et al., Proc. Natl. Acad. Sci. USA, volume 83 (1986) p2934-2938). Hybridised DNA probes are commonly visualised by fluorescence labelling which can give rise to a specific fluorescence signal which identifies a specific chromosome or fragment of chromosome. By marking specific chromosomes, hybridisation probes circumvent the need to identify specific chromosomes on the basis of size and banding patterns. This can simplify the identification of a chromosomal abnormality such as a trisomy whereby three fluorescently marked chromosomes rather than two are immediately visualised without any consideration of size or banding pattern. With probes which hybridise to lengths of chromosomes, identification of translocations are facilitated through the immediate visualisation of a section of fluorescently marked chromosome translocated to an unmarked chromosome.

Hybridisation probes allow individual chromosomes to be identified on the basis of a fluorescent hybridisation signal rather than by size and banding pattern, and it has therefore been possible to use probes to detect chromosomes in non-mitotic (interphase) cells where chromosomes have a diffuse structure which precludes visual identification by staining. With probes specific for centromeric regions of individual chromosomes, the presence of two fluorescent spots in an interphase cell can reliably indicate the presence of a pair of specific chromosome whilst three spots from the same probe can reliably indicate a trisomy. Such chromosome analysis on interphase cells avoids the need to culture cells for accumulation of mitoses and thus greatly reduces the time and skill required for analysis of chromosomal abnormalities. The technology can also be applied for the detection of other chromosome defects such as translocations and deletions using pairs of chromosome probes which span a conserved translocation or deletion site. Probes homologous to chromosomal regions located in close proximity will give rise to hybridisation signals such as fluorescent spots which are in close proximity such that they can be clearly recognised as probes hybridising to the same chromosome fragment. Thus, for translocations such as the 9:22 translocation in chronic myelogonous leukemia (CML), one chromosome probe might hybridise to one side of the translocation breakpoint in chromosome 9 and another chromosome probe hybridise to the other side of the breakpoint. For deletions such as those in Duchenne Muscular Dystrophy (DMD), one chromosome probe might hybridise to one side of the deletion region in the X chromosome and another chromosome probe hybridise to a region frequently deleted in DMD. This will permit the investigator to detect DMD as the absence of one of a pair of fluorescent spots. Thus, the spatial positioning of chromosome probes hybridised to chromosomes in interphase cells can indicate specifically if a lesion such as a translocation or deletion is present. The major limitation to widespread use of current technology for analysis of interphase cells by chromosome probe is that considerable technical skill is required for the use of hybridisation probes. Hence, there is a major need for simpler hybridisation methods, preferably requiring less manipulation and avoiding, if possible, precise pipetting steps.

It is an aim of the present invention to provide a simpler method for performing nucleic acid hybridisation reactions, in particular to provide a method which does not require any precise pipetting steps.

### Summary of the Invention

In one aspect the invention provides a composition for use in nucleic acid hybridisation assays, comprising a labelled nucleic acid hybridisation probe, or a precursor thereof, reversibly bound in desiccated form to a solid support and being releasable therefrom upon the addition of water or an aqueous solution and wherein the hybridisation assay is performed in contact with the surface of the solid support upon which the nucleic acid probe is resersibly bound.

The arrangement of the present invention is preferably one where the probe or precursor thereof is readily releasable from the solid support. "Readily releasable" as herein defined means, for the purposes of the present specification releasable by any aqueous solution.

Nucleic acid probes or precursors may comprise either DNA or RNA. Such probes may be labelled by any of a number of means known to those skilled in the art (e.g. fluorescent labelled, radio-labelled, enzyme labelled). Thus probes or precursors may be either labelled with a reagent giving rise to a directly determinable hybridisation signal or may be labelled with a substance (e.g. biotin) which interacts, after hybridisation, with one or more other substances (e.g. avidin), said interaction giving rise to a determinable hybridisation signal.

Hereinafter, generally references to "probes" are intended also to refer to precursors thereof, where content permits.

Probe precursors are typically unlabelled probes.
Labelling of the precursors to form labelled probes may occur before, during or after hybridisation of the precursor to the sample nucleic acid. Conveniently, labelling of the precursor is achieved by extending the probe, using a polymerase such as DNA polymerase I or Taq polymerase (in a manner well known to those skilled in the art) to add labelled nucleotides.

The solid support may provide a substantially planar surface.

Supports with substantially planar surfaces may conveniently comprise glass or plastics. Preferably the support with a substantially planar surface comprises a thin, translucent material, exemplified by a glass coverslip or microscope slide. Such a preferred embodiment possesses advantages discussed further below.

Where the solid support for the probe comprises glass, it is generally found that the probe will not bind to the support in a manner which is readily reversable upon the addition of water or an aqueous solution, which is a preferred feature of the invention.

This is because nucleic acid comprises many negative charges and therefore binds strongly to glass, which generally comprises many positive charges.

In order to reduce the strength of this electrostatic attraction it is typically necessary to treat the glass to neutralize at least some of its charge. Conveniently this is done by siliconizing the glass, eg by treatment with dimethylchlorosilane, in a manner well-known to those skilled in the art.

Such treatment is not necessary for special types of glass, or for other materials, which do not possess so many positive charges.

The probe is generally applied to the solid support in an aqueous solution and left to dry (either at ambient temperature or at an elevated temperature to speed drying). The probe may be applied in one spot to the surface of the support or more preferably is applied in several spots, forming an array. Alternatively, if it is desired to spread the probe over a hydrophobic surface (e.g. siliconized glass) the probe may be applied in an organic solvent mixture (e.g. 50% methanol/10% acetic acid mixed 3:1 (v/v) with water), or in a detergent solution, such as sodium dodecyl sulphate.

In a particular embodiment, a plurality of different probes may be reversibly bound to a single support. This is especially convenient if using two probes, each of which is labelled with a fluorescent label of a different colour, (e.g. fluorescein and coumarin).

The aqueous solution which may release the reversibly bound probe preferably comprises hybridisation fluid, the exact composition of which is variable depending upon the circumstances. For example, it is well known to those skilled in the art that the composition of hybridisation fluid (especially the concentration of salts therein), together with other factors (e.g. temperature of hybridisation), determines the stringency of hybridisation (i.e. the degree of homology between sequences which have become hybridised). Typically hybridisation fluid comprises an aqueous solution of about 0.3M sodium chloride, and about 0.03M sodium citrate, with the pH adjusted to approximately pH7. Clearly, however, the composition may vary, depending upon the degree of homology between the nucleic acid probe and the nucleic acid in the sample to be assayed; and depending upon the stringency of hybridisation desired. The hybridisation fluid may further comprise optional adjuncts, such as formamide (at about 50% v/v) or dextran sulphate (at about 10% w/v).

It is preferred that the probe precursor is labelled prior to performing the hybridisation assay. However, it will be appreciated that this is not essential: the probe precursor may be labelled during, or after, hybridisation to a sample nucleic acid.

In one embodiment, other substances, in addition to the probe or precursor thereof, may be immobilised (either reversibly or irreversibly) on the same support as the probe/probe precursor or on one or more separate supports. Typically these other substances will interact with the probe or precursor. For example, they may be binding substances (such as antibodies) which bind to the probe or precursor or they may be labelling reagents which label the probe or precursor.

For example, a labelled nucleic acid probe (e.g. derivatised with biotin or fluorescein) might be reversibly immobilised onto one glass coverslip and reagents for further labelling the hybridised probe (e.g. labelled anti-biotin or anti-fluorescein antibodies) might be immobilised onto one or more separate glass coverslips which are applied separately following hybridisation and removal of the first glass coverslip from the hybridised sample.

Alternatively, one may envisage an example where a combination of the nucleic acid probe precursor and reagents for labelling the probe precursor is provided on contiguous solid phase whereby sequential introduction of the nucleic acid probe precursor and the labelling components onto the sample can be achieved.

Clearly, substances in place of or in addition to, labelling reagents may be co-immobilised (reversibly or irreversibly) on the same or a different support as the probe precursor.

For example, a DNA polymerase might be co-immobilised onto a glass coverslip or provided on a second glass coverslip in order to effect the extension of the nucleic acid probe or precursor thereof on a template provided by the sample nucleic acid. In addition, the deoxynucleotides for such an extension might also be immobilised on the same or another coverslip. Thus, hybridisation assays involving extensions from nucleic acid probes or precursors such as the polymerase chain reaction (PCR, US patent nos. 4683195 and 4683202) might be conveniently be undertaken through providing the components of the extension reaction on a solid phase in conjunction with the nucleic acid probes. Of particular interest in the analysis of mammalian chromosomes is the technique of primer in situ labelling (PRINS, see Koch et al., Chromosoma vol. 98 (1989) p259) whereby one or more unlabelled nucleic acids probes are hybridised to a particular region of the sample chromosome preparation and extended using a DNA polymerase to incorporate detectable nucleotides such as biotinylated nucleotides in order to subsequently visualise the site(s) of hybridisation on the chromosome(s). As demonstrated in example 9 below, the PRINS method can be performed by co-immobilising a nucleic acid probe precursor and a DNA polymerase on the same glass coverslip thus providing a particularly convenient means of providing probe and enzyme for performance of the method. Combination of one or more nucleic acid probes and the reagents associated with a particular hybridisation method can be envisaged on a contiguous solid phase whereby sequential introduction of the nucleic acid probe and the associated reagents onto the sample can be achieved.

In another aspect the invention provides a method of performing a nucleic acid hybridisation assay, comprising: treating a labelled nucleic acid probe or precursor thereof, reversibly bound in dessicated form to a solid support and being releasable therefrom upon the addition of water or an aqueous solution, by the addition of water or an aqueous solution to release the probe or precursor; bringing the released probe or precursor into contact with a sample to be assayed, comprising nucleic acid, under conditions which allow the hybridisation of homologous or substantially homologous sequences; if necessary, treating the precursor in suitable manner to produce a labelled probe; removing unhybridised probe from the mixture and determining the presence (if any) of labelled probe hybridised to the sample nucleic acid, wherein the assay is performed in contact with the surface of the solid support.

An advantage of this method over prior art techniques is that the use of a reversibly bound probe in dessicated form tends to reduce the need for precise pipetting steps, allowing the assay to be performed by relatively unskilled personnel, relatively quickly and simply.

The method may be used for both qualitative and quantitative determinations.

In a preferred embodiment the probe is released and brought into contact with the sample to be assayed in a single step by the simple addition of water or an aqueous solution. Typically an aqueous solution is used which comprises hybridisation fluid as discussed above.

Generally unhybridised probe is removed from the hybridisation assay mixture by performing one or more washing steps. Typically the washing fluid comprises hybridisation fluid, the composition-of which is slightly variable, as detailed previously.

The assay is performed under conditions such that the nucleic acid hybridisation probe will hybridise to homologous or substantially homologous sequences. A substantially homologous sequence is one which is not entirely complementary to the probe, but nearly so. Typically such substantially homologous sequences will share at least 90% homology.

It is a preferred feature of the invention that, particularly when using fluorescent-labelled probes, the probes are reversibly bound to a solid support comprising a thin, translucent material such as glass or plastic which comprises a substantially planar surface, such material typically comprising a microscope slide or coverslip. This arrangement has a number of advantages.

Firstly, the microscope slide or coverslip to which the probe is reversibly bound may be placed in close proximity to a surface of the material (typically a coverslip or microscope slide) supporting the sample to be assayed such that the hybridisation fluid used to release the probe may be drawn between the two surfaces by capillary action. This has the advantage that the person performing the assay need not precisely measure the quantity of any reagent. Furthermore, if both the material supporting the probe and the material supporting the sample to be assayed are translucent, one may readily determine the presence of hybridised fluorescent-labelled probe by employing fluorescence microscopy. Naturally however, the method of determining the presence of hybridised probe depends upon the manner in which the probe has been labelled.

It will be readily apparent to those skilled in the art that the method defined above is particularly well-suited to analysis (particularly chromosome analysis) of mammalian cells, especially human cells, including the analysis of fresh blood cells or fresh amniotic cells, and to the analysis of sperm cells, chorionic villus cells and umbilical cord blood cells.

Further, it will be appreciated that the method defined above could be applied to analysis of several different chromosomal characteristics. For example, probes homologous to a specific chromosome can provide information on copy number of that chromosome, for example to determine the presence of a trisomy. Probes which span a translocation or deletion breakpoint can provide additional spatial information to indicate whether a chromosome is normal (2 hybridisation signals juxtaposed) or abnormal, such as in a translocation (one hybridisation signal dissociated from the other), or in a deletion (one of a pair of two adjacent hybridisation signals missing).

The method of the invention is clearly not restricted to freshly prepared cells.

For example, it has been discovered that the method of the invention can be equally applied to both cultured mammalian cells and to freshly isolated mammalian cells, although some minor modifications to the methodology are required for certain cell types in order to optimise the detection of chromosomes with probes. In particular, the degree of permeability to nucleic acids probes achieved by incubation in hypotonic solutions varies between cell types, and it has been found that certain cell types including amniocytes, require pretreatment with RNAse or proteinase or both in order to remove cellular components to which probes may bind.

It has also been found that the method of the invention will also apply to "archive" material such as cytogenetic slides analysed previously by staining which can, at some later time, be subjected to hybridisation with chromosome probes to yield chromosome-specific hybridisation signals. It will be appreciated that such a method, with minor modifications, may be applied to many other types of cell preparations such as tissue sections and to material of a variety of ages subject to the presence of intact undegraded chromosomal material.

It will also be apparent to those skilled in the art that the method of the invention, whilst particularly suited to the chromosome analysis of mammalian cells, is not limited to this application.

For example, it may be applied to most types of mammalian cells, to micro-organisms containing nucleic acid, and to various preparations of the aforementioned.

The method may be applied, for example, to the detection of virus-infected mammalian or bacterial cells, to the detection of eukaryotes (such as yeast, fungi, protozoa, algae or plant cells) or to the detection of prokaryotes.

In particular, the method of the invention may find application in the detection of micro-organisms (e.g. viruses, bacteria, rickettsiae), among samples of mammalian cells (whether cultured or freshly prepared) or among samples of cultures of other micro-organisms (such as bacteria, fungi or yeasts).

In another aspect the invention provides a kit for use in nucleic acid hybridisation assays, comprising: a labelled nucleic acid hybridisation probe or precursor thereof reversibly bound in dessicated form to a solid support and being readily releasable therefrom upon the addition of water or an aqueous solution, for performance of the assay in contact with the surface of the solid support; and instructions for use.

The kit may also comprise other substances bound in dessicated form to the same or a further solid support. The said other substances may be bound reversibly or irreversibly.

Conveniently, where the kit comprises a probe precursor the kit will comprise other substances bound in dessicated form to a solid support which may act as labelling reagents.

Typically the nucleic acid hybridisation probe will be reversibly bound to a coverslip or microscope slide. It is a preferred feature therefore that the kit further comprises vulcanising solution (of a composition well known to those skilled in the art). Other preferred components of the kit include: hybridisation solution (preferably of a composition carefully matched to create the ideal conditions of stringency of hybridisation of the probe with the intended target sequence in the sample to be assayed); washing solution and an anti-fade solution.

The signal from fluorophores (such as are used to fluorescently label probes) tends to be quenched with time during excitation of the fluorophore such as is experienced in fluorescence microscopy. Anti-fade solution, well known to those skilled in the art, helps to reduce this tendency. Preferably, the anti-fade solution comprises propidium iodide, which tends to stain cell nuclei red, and may therefore act as a counterstain to certain commonly-used fluorophores (e.g. fluorescein).

The kit may also comprise microscope slides which may have been treated, in a manner known to those skilled in the art, to improve results obtained when analysing amniocytes (such as are obtained from anmiocentesis).

The various aspects of the invention will be better understood by reference to the following illustrative examples and drawings, of which:
Figure 1 is a schematic representation of nucleic acid hybridisation probes reversibly immobilised upon different solid supports;
Figure 2 is a photomicrograph illustrating results obtained from hybridisation assays using fluorescent-labelled probes specific for a particular human chromosome; and
Figure 3 is a sketched representation of a photomicrograph which might be obtained from hybridisation assays using fluorescent-labelled probes specific for two loci of a particular human chromosome.

### Detailed Description

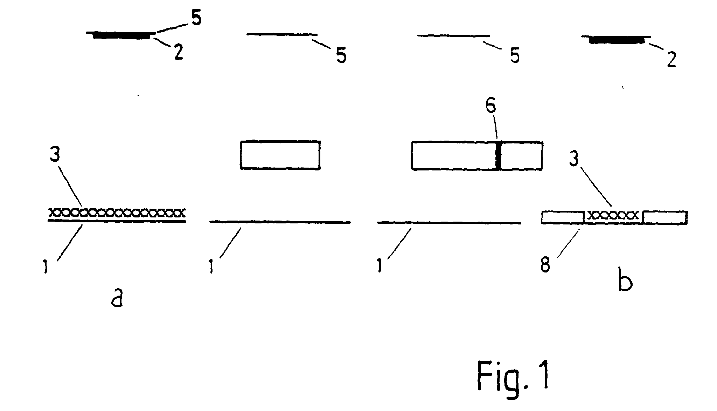

Figure 1 shows examples of the manner in which probes may be reversibly immobilised upon solid supports. Figure la shows a probe 2 reversibly immobilised upon one surface of a conventional microscope slide coverslip 5, with substantially planar surfaces. The coverslip is then positioned such that the side coated with the probe contacts the surface of a conventional microscope slide 1 which is supporting the sample to be assayed 3. An appropriate amount of hybridisation fluid is then placed on the slide and drawn under the coverslip by capillary action, thereby releasing the probe from the coverslip and allowing it to hybridise with any homologous sequences in the sample to be assayed. An equally effective method is to place hybridisation fluid directly on top of the sample to be assayed and then position the coverslip on top of the hybridisation fluid, which spreads beneath the coverslip by capillary action.

In an alternative arrangement, shown in Figure 1b, the surface of the coverslip coated with probe 2 may be used in conjunction with a microscope slide 8 comprising a well for retaining fluid. The sample to be assayed is applied to the well, together with hybridisation fluid and the probe-coated surface of the coverslip positioned such that it contacts the hybridisation fluid and the probe is released.

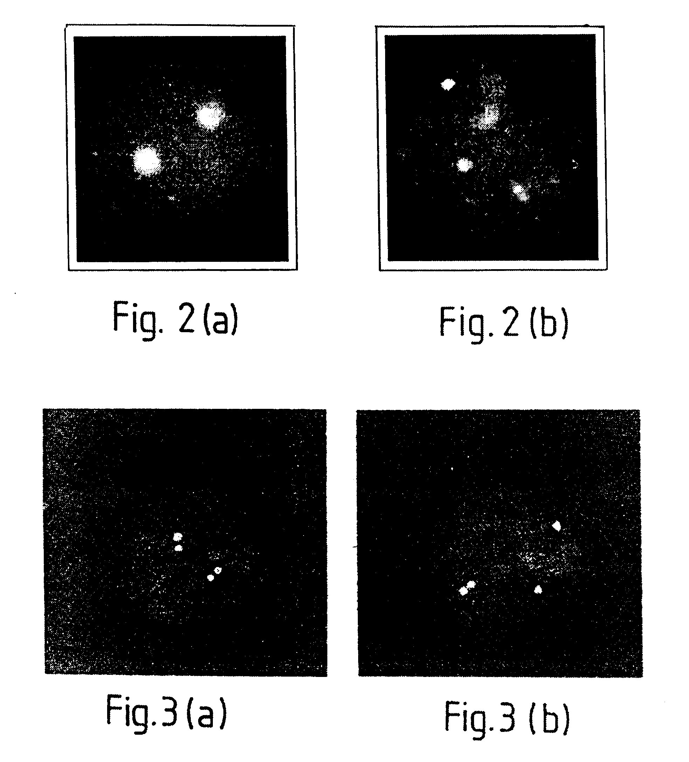

Figure 2 is described with reference to Example 1 below.

Figure 3 is a sketch of hypothetical photomicrographs that might be obtained using a pair of fluorescent-labelled probes specific for either side of the translocation breakpoint on human chromosome 9, which translocation leads to the association of chromosomes 9 and 22 in patients with chronic myelogonous leukemia (CML). Figure 3a shows the results which would be obtained from hybridisation in a normal interphase cell: the pairs of probes hybridise adjacently to each copy of chromosome 9, showing no translocation has occurred. Figure 3b shows the results which would be obtained from hybridisation with an abnormal interphase cell from a CML patient: the pair of probes has hybridised adjacently to one copy of chromosome 9 but have also hybridised at two different loci, showing that a translocation has occurred in one copy of chromosome 9, leading to physical separation of the two hybridisation loci.

### Example 1

This example relates to the detection of cells trisomic for chromosome 12 in chronic lymphocytic leukemia (CLL). 0.1ml of whole blood from either a healthy volunteer or a CLL patient were cultured in 5ml of medium comprising RPMI 1640 (Gibco, Paisley, UK), 10% foetal calf serum (Gibco®) and 0.05ml of phytohaemagglutinin solution (Gibco®, M-form). Cells were incubated for 72 hours at 37°C and 0.05ml colchicine (Sigma, Poole, UK) was added. After incubation for a further 60 minutes, cells were centrifuged and the pellets were resuspended in 0.075M potassium chloride solution. After 10 minutes, cells were again centrifuged and the pellets resuspended in methanol:glacial acetic acid 5:1(v/v). The cells were further pelleted and resuspended 3 times in the methanol:acetic acid fixative solution. A drop of cells at a minimum concentration of 10⁴ per ml was placed on several microscope slides and left to dry at room temperature. Slides were then incubated at 65°C on a hot-plate for 1 hour and stored at -20°C. Just prior to hybridisation, slides were immersed sequentially in cold 70% ethanol for 2 minutes, cold 85% ethanol for 2 minutes and finally cold 100% ethanol for 2 minutes and dried.

For hybridisation, a DNA probe homologous to the centromeric region of chromosome 12 was used. 25µg of plasmid p-alpha-12H8 (Looyenga et al., Cytogenet. Cell Genet. 54 (1990) p216) was nick translated by digestion at 37°C for 10 minutes with 0.005 units DNAse I (Life Technologies, Paisley, UK) in 25µl of 40mM Tris. HCl pH8 and 6mM MgCl₂. The reaction was stopped by heating to 70°C for 10 minutes. 3µl of digested DNA was then mixed with 5µl of 10xA4 buffer (200µM deoxyadenosine triphosphate, 200µM deoxycytosine triphosphate, 0.5M Tris.HCl pH7.2, 0.2mM MgCl₂, 0.1mM beta-mercaptoethanol, 0.1mg/ml bovine serum albumin (fraction V, Sigma®), 3.3µl bio-11 dUTP (from 100µg/ml stock, Sigma®) and water to 49µl. 1µl DNA polymerase I was added and the mixture incubated at 15°C for 1.5 hours. The reaction was stopped by addition of 5µl 0.3M EDTA pH8. In some experiments, 2µl of probe was mixed with 100µl methanol:glacial acetic acid 3:1 (v/v) and this was dropped onto the unmarked side of a siliconised 25mm² microscope slide coverslip (Number 1 coverslip, BDH, Dorset, UK). A second siliconised coverslip was placed onto the probe mixture via the unmarked side in order to evenly spread the probe and the sandwich of probe with coverslips was incubated for 2 hours at 37°C. The dry coverslips were then separated and both used for hybridisation. In other experiments, the probe diluted into 200µl of water was either dotted as individual aliquots of 5µl in an array over the coverslip or was manually spread over the coverslip using a siliconised glass rod. In each of these cases, the probe was dried onto the coverslip at 37°C.

Hybridisation was undertaken by adding a coverslip onto a spot of hybridisation buffer (50% formamide, 0.3M sodium chloride, 0.03M sodium citrate pH7, 10% w/v dextran sulphate (molecular weight average 500,000 daltons, Sigma®) onto each microscope slide. Coverslips were sealed at the edges with rubber solution and the slides heated to 85°C for 10 minutes on a heated block. Slides were then incubated at 37°C overnight. Coverslips were then removed and washed three times for 5 minutes at 45°C in 50% formamide, 0.3M sodium chloride and 0.03M sodium citrate pH7. Slides were then washed twice for 5 minutes in PN buffer (0.1M sodium phosphate buffer pH8, 0.1% NP40) at 45°C and room temperature respectively. Slides were then incubated in the dark at room temperature for 20 minutes in 5µg/ml fluorescein-avidin DCS solution (Vector Laboratories, Peterborough, UK) in PMN buffer (0.1M sodium phosphate buffer pH8, 0.1%NP40, 0.02% sodium azide and 5% non-fat dried milk (Marvel™, Cadbury, Birmingham, UK)). Slides were washed three times in the dark in PN buffer and further incubated in the dark for 20 minutes in 5µg/ml biotinylated goat anti-avidin (Vector Laboratories) in PMN buffer. Slides were again washed three times in the dark in PN buffer, excess buffer was removed and the slides were stained in 1µg/ml propidium iodide (Sigma®) in glycerol containing 250µg/ml 1,4-diazabicyclooctane (DABCO, Aldrich, Dorset, UK). A coverslip was added to each slide prior to microscopy.

Typical results are shown in Figure 2.

For the majority of blood cells from a normal volunteer (mainly megakaryocytes), two fluorescent spots were observed (Figure 2a) whilst the majority of blood cells from the CLL patient displayed three fluorescent spots (Figure 2b) corresponding to a trisomic chromosome 12.

### Example 2

The method of example 1 was repeated using either a centromeric chromosome 12 probe or, alternatively, a centromeric chromosome 1 probe derived from plasmid pUCL77 (Cooke and Hindley, Nucleic Acids Research 6 (1979) p3177). As an alternative to culturing of blood cells, chromosome analyses were undertaken directly on both amniotic fluid and fresh blood. In order to achieve a pair of fluorescent spots from normal amniocytes and normal blood cells, cells were centrifuged and cell pellets were resuspended in 0.075M potassium chloride for 30 to 60 minutes. Cells were then centrifuged and resuspended in methanol:glacial acetic acid 3:1(v/v) and were then applied to microscope slides. Cells on the slide were treated for 1 hour at 37°C in 100µg/ml RNAse A (bovine RNAse, Boehringer, Lewes, UK). Slides were then washed four times in 0.3M sodium chloride, 0.03M sodium citrate pH7. Slides were then washed successively in 70% ethanol, 85% ethanol and 100% ethanol. Slides were then incubated for 30 minutes at 37°C in 0.5µg/ml proteinase K (Sigma®), centrifuged and washed twice in 0.3M sodium chloride, 0.03M sodium citrate pH7. Slides were then resuspended in 4% paraformaldehyde solution, centrifuged and washed twice in 0.3M sodium chloride, 0.03M sodium citrate pH7. Slides were then washed successively in 70% ethanol, 85% ethanol and 100% ethanol and hybridised with DNA probe as above.

For the majority of either fresh (uncultured) amniocytes or blood cells, two fluorescent spots were observed with either the chromosome 12 or chromosome 1 DNA probes.

### Example 3

A number of steps from the methods of examples 1 and 2 were eliminated through the use of directly fluorescein-labelled probe. Probe DNA was nick-translated as detailed in example 1 except that instead of bio-11 dUTP, fluorescein-deoxyuridine triphosphate (Amersham International, Little Chalfont, UK) was used at 20µM final concentration, deoxythymidine triphosphate was used at 20µM final concentration and the other deoxyribonucleotide triphosphates were used at 60µM final concentration with the incubation at 15°C extended to 3 hours. Following the hybridisation incubation and washing in PN buffer, slides were treated directly with 1µg/ml propidium iodide in DABCO and viewed under the microscope. Fluorescent spots were observed as for examples 1 and 2.

### Example 4

The application of the method in examples 1 to 3 to "archive" material was investigated by analysis of 3 week-old slides. A slide comprising fixed blood cells was washed in phosphate-buffered saline and then in 100% ethanol and dried. Slides were then incubated at 65°C on a hot-plate for 1 hour and stored at -20°C.
Hybridisations were carried out as above with a result that the centromeric chromosome 12 probe from p-alpha-12H8 gave rise to pairs of fluorescent spots in the majority of blood cells.

### Example 5

The method outlined in example 3 is here further described with reference to its application to the analysis of germ cells. In particular, this example relates to the detection of cells disomic for chromosome 1 from patients who may have been exposed to radiation. A directly labelled chromosome 1 probe (plasmid pUC1.77 -see example 2 above) was used. Sperm samples were donated from a healthy volunteer and also from a radiation-exposed patient.

The sperm samples were suspended in 10ml phosphate buffered saline (PBS-0.14M sodium chloride, 0.002M potassium chloride, 0.01M disodium hydrogen orthophosphate, 0.001M potassium dihydrogen orthophosphate pH7.4) mixed and pelleted by centrifugation. After careful removal of supernatant, fresh fixative (methanol:acetic acid 3:1 (v/v)) was added until a 'milky' solution was obtained. Fixed cells were then applied to microscope slides and allowed to dry. Cells on the slide were washed in PBS for 5 minutes, dehydrated successively in 70% ethanol, 85% ethanol and 100% ethanol prior to treatment in 0.1M DTT/1% trypsin solution for 12 minutes. After a further successive dehydration series, samples were hybridised with DNA probes as described above. Typical results are shown in Figure 4. For the majority of cells from the healthy volunteer, a single fluorescent spot was observed with the chromosome 1 probe. However, a small number of cells contained 2 fluorescent spots indicating a small proportion of disomy 1 cells in the cells from the radiation exposed patient.

### Example 6

This example relates to the prenatal detection of trisomy 18 in a chorionic villus sample (CVS). A probe homologous to the centromeric region of chromosome 18 was used (Devilee et al. Nucleic Acids Research 14:2059-2073, 1986). The probe was prepared essentially as described in example 3 above.

Chorionic villus cells were prepared by incubation of dissected villi in 2ml Chang medium (Metachem Diagnostics Ltd, Northampton, England) containing Dispase (4mg/ml) for 2 hours. Colchicine (0.01% w/v) was added and further incubated for 1 hour prior to centrifugation. The resulting cell pellet was resuspended in 5ml 1% (w/v) sodium citrate, incubated for 15 minutes at 37°C cells pelleted by centrifugation and the resulting cell pellet was washed twice in ice cold fixative (methanol:acetic acid (3:1) v/v). The fixed cells were then applied to glass slides prior to sequential dehydration through an ethanol series (70%, 85% and 100%). Following the hybridisation of the fluorescein labelled probe and post hybridisation stringency washes, slides were treated as described in example 3 above. For the majority of CVS cells, three cluorescent spots were observed consistent with trisomy 18 (Edwards Syndrome).

### Example 7

This example relates to the determination of foetal sex from umbilical cord blood by utilisation of two colour fluorescence in situ hybridisation. 10µl of cord blood was added slowly with mixing to 100µl of fresh fixative (methanol:acetic acid (3:1) v/v). The fixed cells were then applied to glass slides prior to sequential dehydration through an ethanol series (70%, 85% and 100%) prior to hybridisation. The probes are derived from the heterochromatin region of chromosome Y and the centromeric region of chromosome X (Nakahori et al., Nucleic Acids Research 14:7569-7580, (1986) and Willard et al., Nucleic Acids Research 11:2017, (1983) respectively). The probes were labelled essentially as described in example 3 except that the fluorophore used to label the Y probe was Hydroxycoumarin 6-dUTP (Boehringer Mannhein GmbH, Germany).

Following hybridisation incubation and stringency washing, slides were treated directly with 0.7µg/ml propidium iodide in Vectashield (Vector Labs Inc, Burlingame, USA) and viewed under the epifluorescence microscope utilising a dual filter block (Nikon, Japan) for fluorescein and coumarin.

One yellow spot and one blue spot were observed in the fixed lymphocytes indicating the blood to be derived from a male foetus.

The above examples are by no means an exhaustive list of types of samples to which this technology may be applied, but give an indication of scope.

### Example 8

This example relates to the analysis of human amniocytes and peripheral blood cells for chromosomes 13/21, 18, X and Y and utilises kits commercially available based on the method of the invention. "Chromoprobe" kits for the analysis of chromosomes 13/21, 18, X and Y were obtained from Cytocell, Lewknor, UK and were used according to the manufacturer's instructions including the use of ancillary reagents. The frequency of appearance of fluorescence spots in interphase cells from samples of known chromosome complement was measured and the results were shown in the following tables, wherein Tables 1a and 1b show the results obtained from amniocytes and peripheral blood cells respectively using probes specific for chromosomes 13/21, Tables 2a and 2b show the results for the same cell types (i.e. amniocytes and peripheral blood cells respectively) using a probe specific for Chromosome 18, Tables 3a and 3b show the results for the same cell types using a probe specific for the X chromosome and Tables 4a and 4b show the results for the same cell types using a probe specific for the Y chromosome.

### Example 9

This example relates to the detection of cells trisomic for chromosome 12 in chronic lymphocytic leukemia (CLL) by a variation to the method of example 1. Cells from a CLL patient were prepared and deposited onto microscope slides as described in example 1. 2µg of the plasmid p-alpha-12H8 (example 1) was digested with the enzyme DdeI used according to the manufacturer's instructions (Promega, Southampton, UK). The digested plasmid DNA was phenol:chloroform extracted and ethanol precipitated (see "Molecular Cloning, A Laboratory Manual", eds. Sambrook, Fritsch and Maniatis, Cold Spring Harbor Press (1989)) and resuspended in 10µl water. Individual 1µl aliquots of this DNA were applied in a 3 x 3 matrix onto a siliconised 25mm² coverslip and allowed to dry. 1 unit of Taq polymerase (Promega) was diluted to 10µl in reaction buffer as recommended by the manufacturer and was applied as 1µl aliquots (3 x 3 matrix) to the same coverslip and allowed to dry.

For hybridisation and extension, 10µl of an aqueous solution of 50µM each of dATP, dGTP and dCTP and 25mM of fluorescein-12-dUTP (Boehringer®) was applied to the sample on the slide and the coverslip containing the digested plasmid and taq polymerase was applied. The slide was heated to 93°C for 5 minutes and then incubated at 70°C for 1 hour. The coverslip was then removed and slide washed at 70°C for 5 minutes in 500mM NaCl and 50mM EDTA, pH8. Slides were then stained with propidium iodide and viewed as in example 1. For the majority of blood cells from the CLL patient, three fluorescent spots were observed corresponding to the presence of three copies of chromosome 12.

The above examples are by no means an exhaustive list of types of samples to which this technology may be applied, but give an indication of scope.

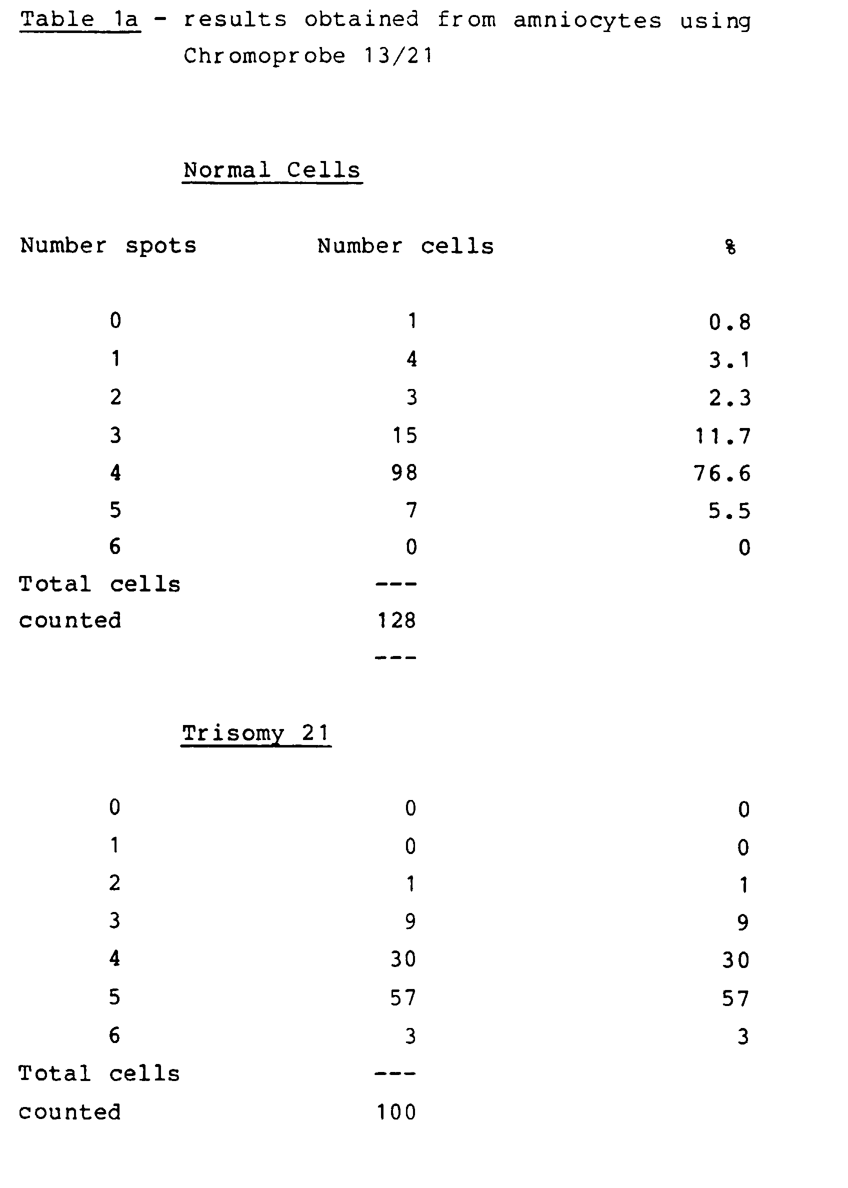

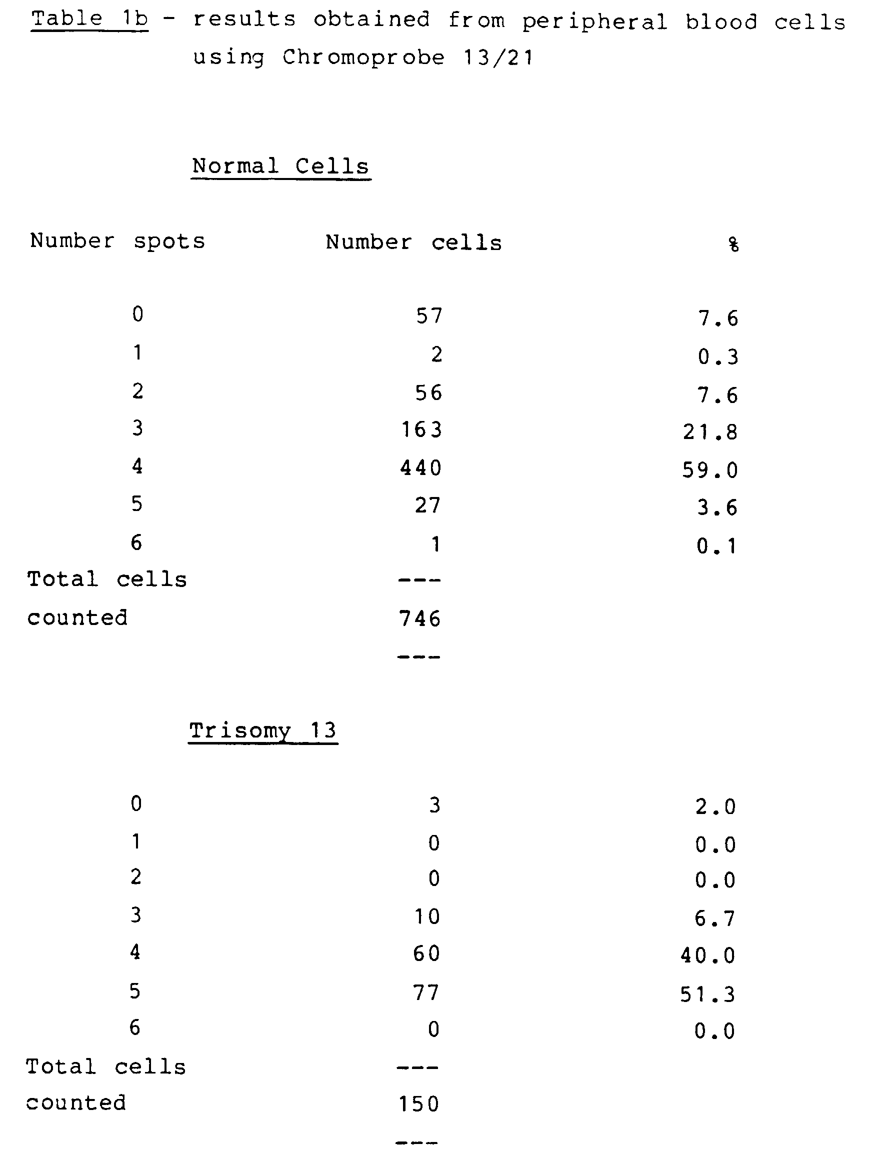

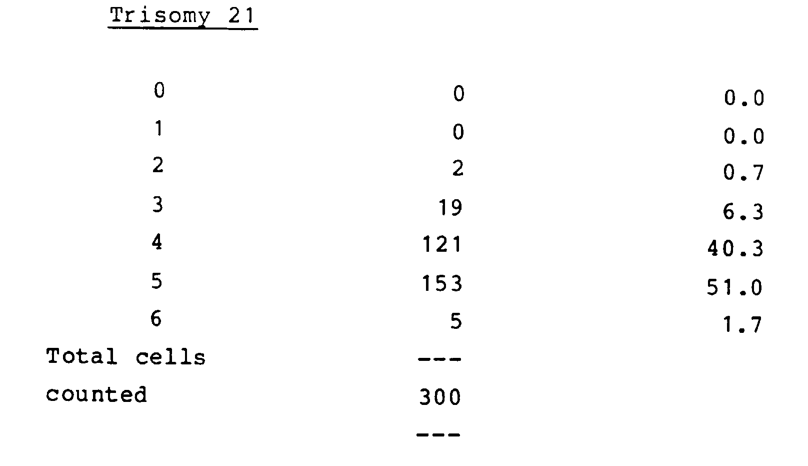

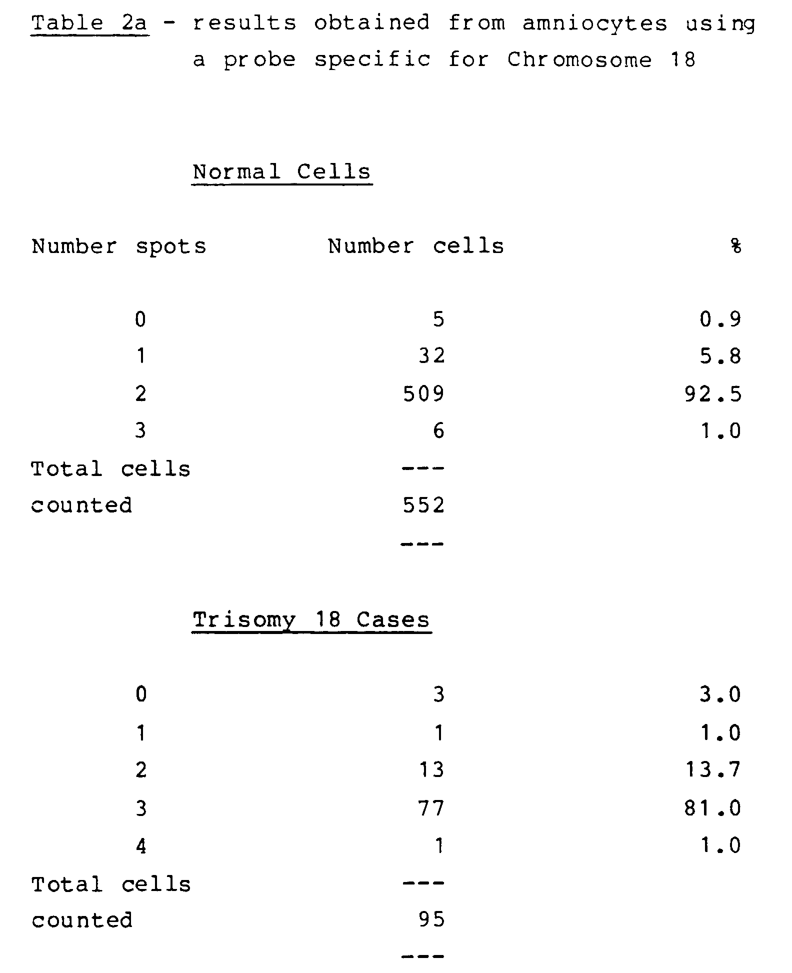

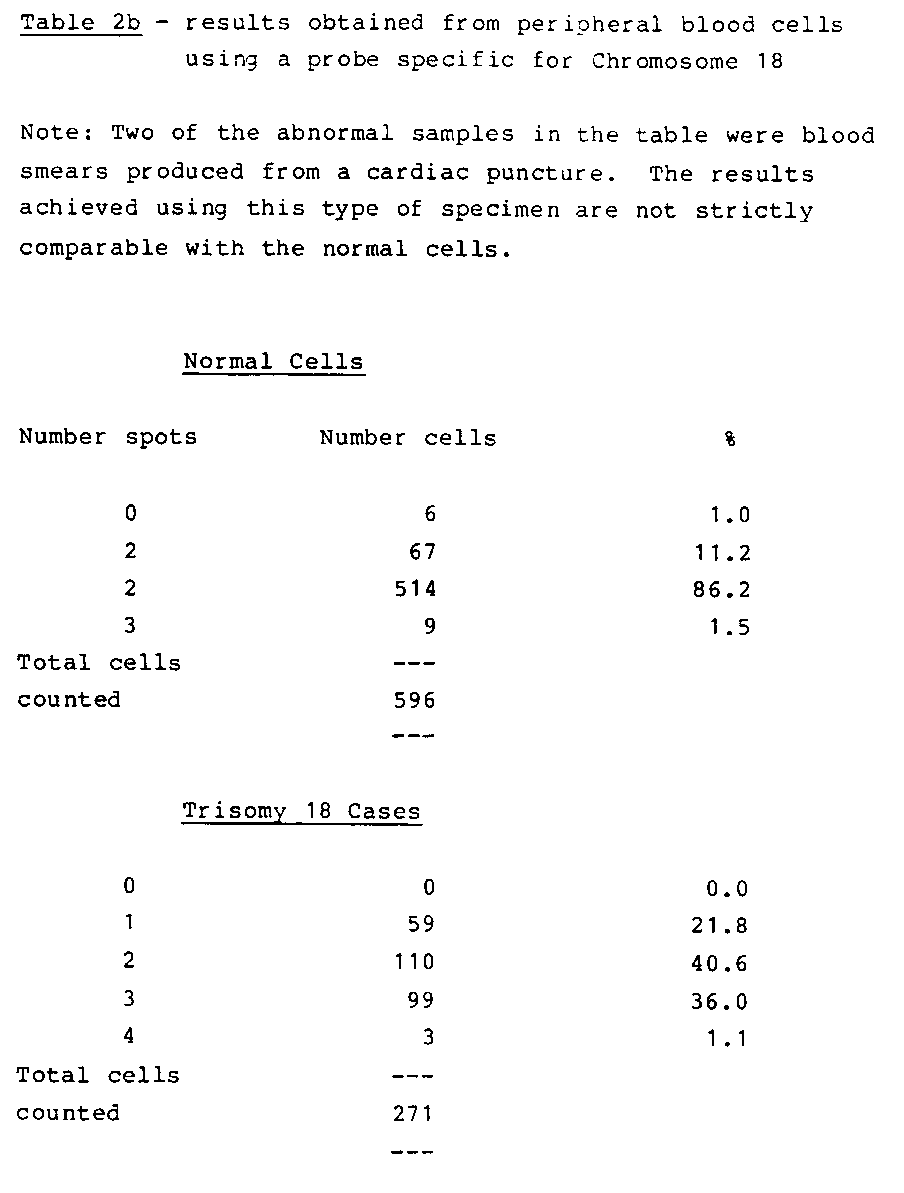

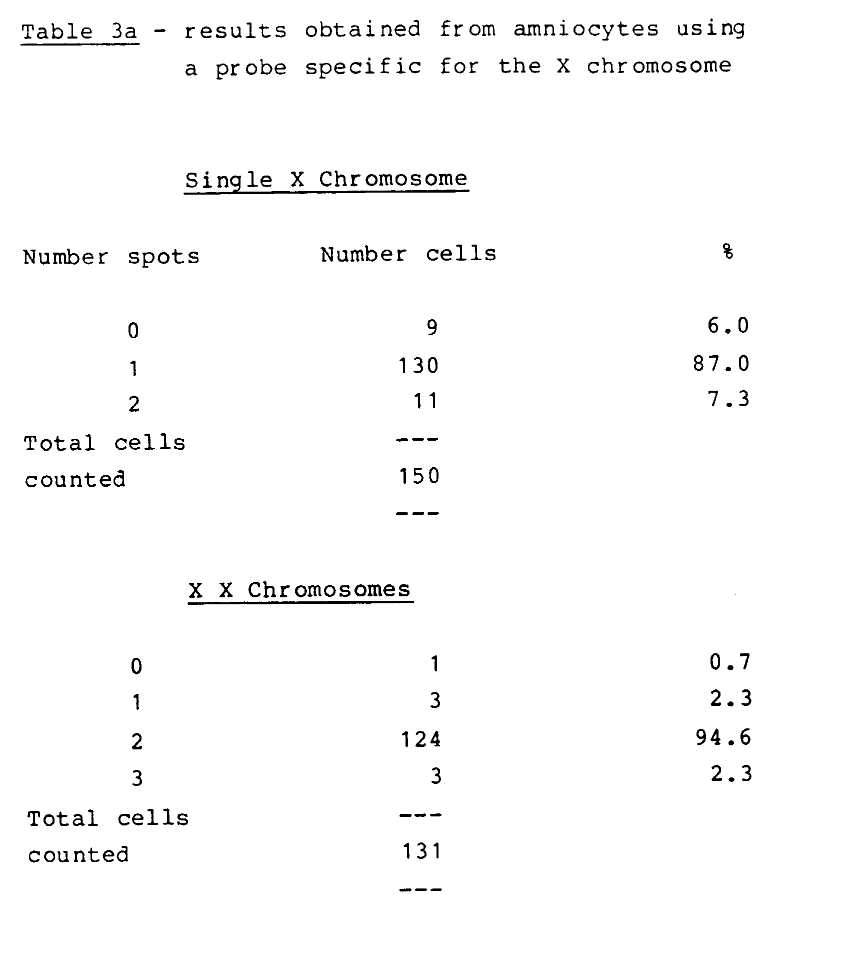

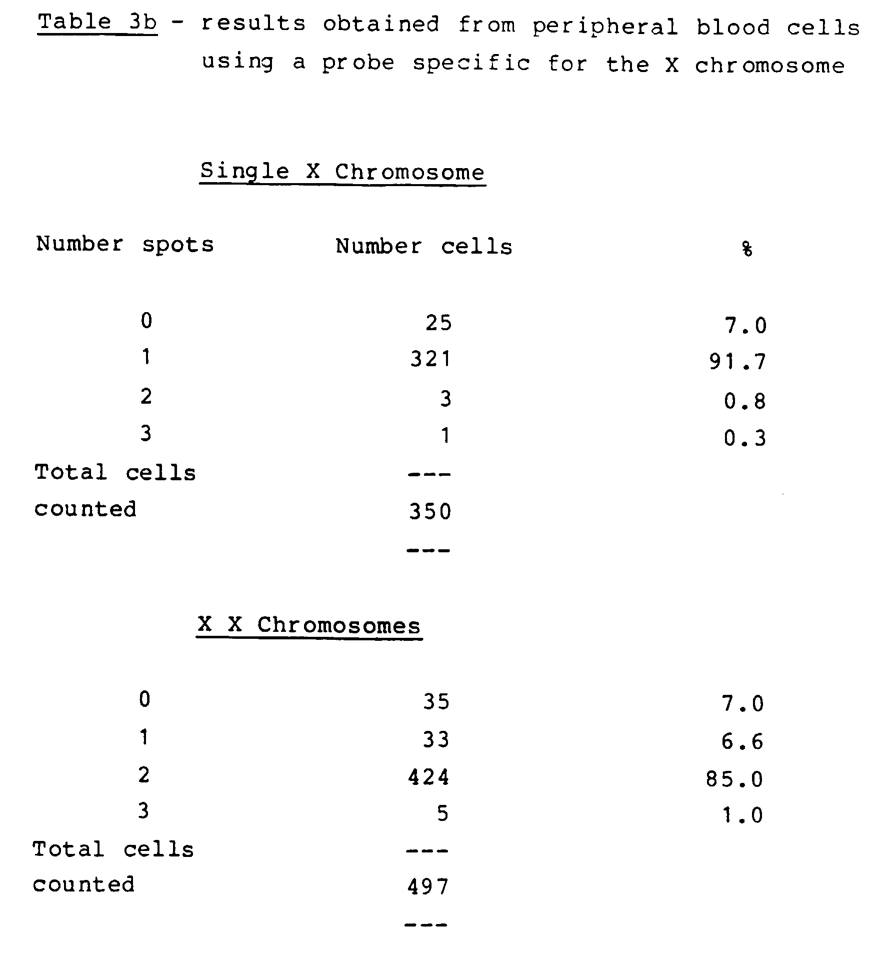

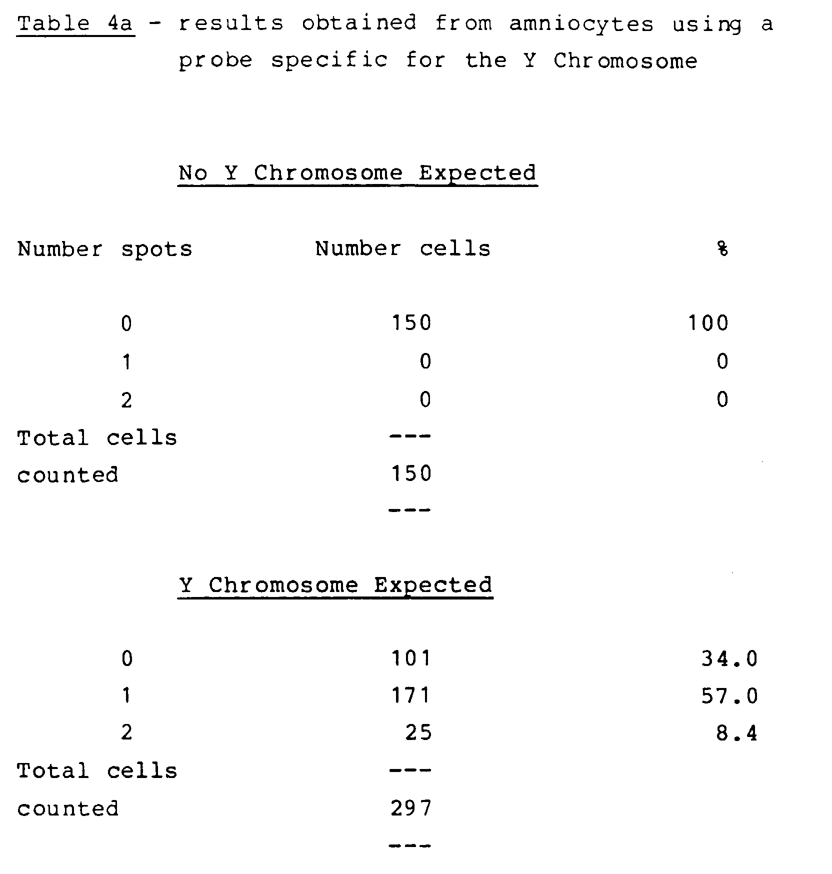

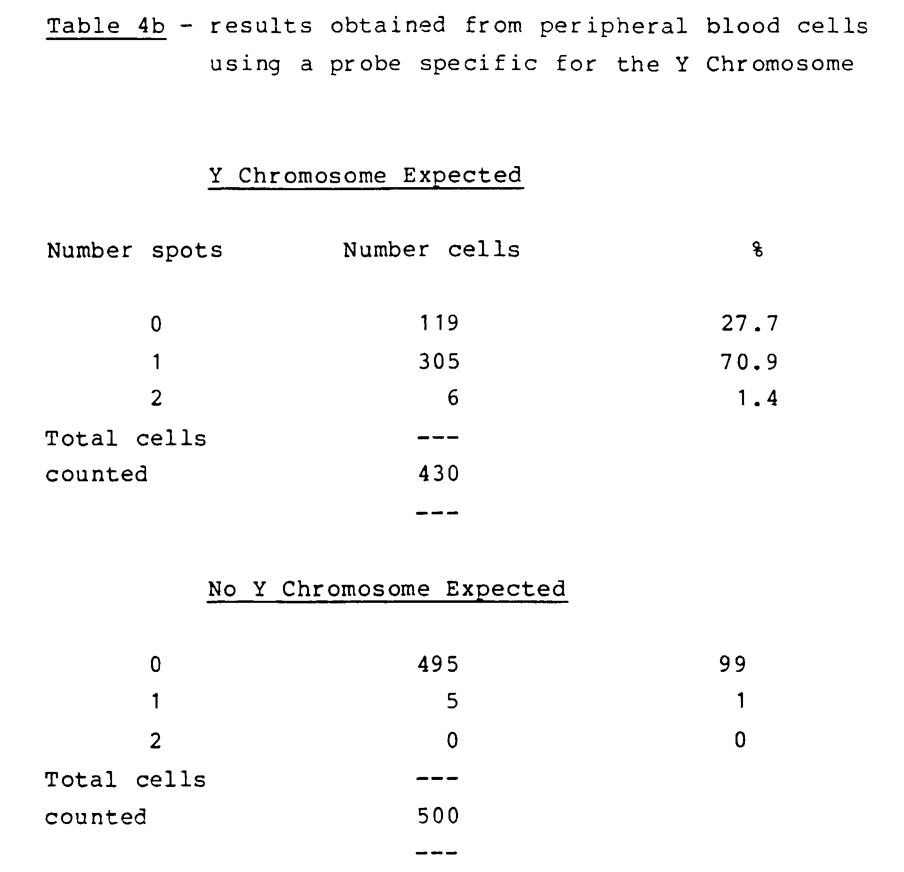

## Claims

1. A composition for use in nucleic acid hybridisation assays, comprising a labelled nucleic acid hybridisation probe, or a precursor thereof, reversibly bound in dessicated form to a solid support and being releasable therefrom upon the addition of water or an aqueous solution, and wherein the hybridisation assay is performed in contact with the surface of the solid support upon which the nucleic acid probe is reversibly bound.

2. A composition according to claim 1, comprising a probe precursor in the form of an unlabelled nucleic acid probe.

3. A composition according to claim 2, wherein labelling of the probe precursor is achieved by the addition thereto of labelled nucleotides.

4. A composition according to any one of claims 1-3, wherein the solid support is substantially planar.

5. A composition according to any one of the preceding claims, wherein the solid support comprises a microscope slide or coverslip.

6. A composition according to any one of the preceding claims. wherein the solid support comprises siliconized glass.

7. A composition according to any one of the preceding claims, wherein a plurality of different probes or precursors thereof are reversibly bound to a single solid support.

8. A composition according to any one of the preceding claims, further comprising other substances bound in dessicated form to the same or a further solid support.

9. A composition according to claim 8. wherein said other substances comprise a reversibly or irreversibly immobilised substance capable of interacting with the nucleic acid probe.

10. A composition according to claim 8 or 9, wherein said other substances comprise an enzyme.

11. A composition according to any one of claims 8, 9 or 10, wherein said other substances comprise a DNA polymerase.

12. A composition according to any one of claims 8-11, wherein said other substances comprise deoxynucleotides for addition to the probe substance.

13. A composition according to any one of claims 8-12, wherein said other substances comprise labelling reagents.

14. A method of performing a nucleic acid hybridisation assay, comprising: treating a labelled nucleic acid probe or precursor thereof, reversibly bound in dessicated form to a solid support and being releasable therefrom upon the addition of water or an aqueous solution, by the addition of water or an aqueous solution to release the probe or precursor; bringing the released probe or precursor into contact with a sample to be assayed, comprising nucleic acid, under conditions which allow the hybridisation of homologous or substantially homologous sequences; if necessary, treating the precursor in suitable manner to produce a labelled probe, removing unhybridised probe from the mixture and determining the presence (if any) of labelled probe hybridised to the sample nucleic acid, wherein the assay, is performed in contact with the surface of the solid support.

15. A method according to claim 14, wherein the probe or precursor thereof is released by the addition of hybridisation fluid.

16. A method according to claim 14 or 15, wherein a precursor reversibly bound to the support is treated in a suitable manner to produce a labelled probe by the use of a polymerase to add labelled nucleotides to the precursor.

17. A method according to any one of claims 14-16, wherein the method is performed in a manner which does not require additional precisely measured quantities of reagents.

18. A method according to any one of claims 14-17, wherein the method is applied to the chromosome analysis of cells.

19. A kit for use in nucleic acid hybridisation assays, comprising: a labelled nucleic acid probe or precursor thereof reversibly bound in dessicated form to a solid support and being releasable therefrom upon the addition of water or an aqueous solution, for performance of the assay in contact with the surface of the solid support; and instructions for use.

20. A kit according to claim 19, further comprising other substances bound in dessicated form to the same or a further solid support.

21. A kit according to claim 19 or 20, wherein said other substances comprise labelling reagents.

22. A kit according to any one of claims 19-21, further comprising one or more of the following: hybridisation fluid; washing solution; vulcanising solution; and anti-fade solution comprising propidium iodide.

## Patentansprüche

1. Zusammensetzung zur Verwendung in Nukleinsäurehybridisierungs-Assays, umfassend eine markierte Nukleinsäureprobe zur Hybridisierung oder einen Precursor dieser, die bzw. der reversibel in getrockneter Form an einen festen Träger gebunden und durch Zugabe von Wasser oder einer wäßrigen Lösung von diesem ablösbar ist, wobei das Hybridisierungs-Assay unter Kontakt mit der Oberfläche des festen Trägers, an den die Nukleinsäureprobe reversibel gebunden ist, durchgeführt wird.

2. Zusammensetzung nach Anspruch 1, umfassend einer Precursorprobe in Form einer unmarkierten Nukleinsäureprobe.

3. Zusammensetzung nach Anspruch 2, worin das Markieren der Precursorprobe durch Zugabe von markierten Nukleotiden zu dieser erreicht wird.

4. Zusammensetzung nach einem der Ansprüche 1 bis 3, worin der feste Träger im wesentlichen planar ist.

5. Zusammensetzung nach einem der vorhergehenden Ansprüche, worin der feste Träger einen Objektträger oder ein Deckglas umfaßt.

6. Zusammensetzung nach einem der vorhergehenden Ansprüche, worin der feste Träger siliconisiertes Glas umfaßt.

7. Zusammensetzung nach einem der vorhergehenden Ansprüche, worin eine Vielzahl von verschiedenen Proben oder deren Precursor reversibel an einen einzigen festen Träger gebunden sind.

8. Zusammensetzung nach einem der vorhergehenden Ansprüche, darüber hinaus umfassend andere Substanzen, die in getrockneter Form an denselben oder einen weiteren festen Träger gebunden sind.

9. Zusammensetzung nach Anspruch 8, worin die anderen Substanzen eine reversibel oder irreversibel immobilisierte Substanz umfassen, die dazu in der Lage ist, mit der Nukleinsäureprobe in Wechselwirkung zu treten.

10. Zusammensetzung nach Anspruch 8 oder 9, worin die anderen Substanzen ein Enzym umfassen.

11. Zusammensetzung nach einem der Ansprüche 8, 9 oder 10, worin die anderen Substanzen eine DNA-Polymerase umfassen.

12. Zusammensetzung nach einem der Ansprüche 8 bis 11, worin die anderen Substanzen Deoxynukleotide zur Zugabe zu der Probensubstanz umfassen.

13. Zusammensetzung nach einem der Ansprüche 8 bis 12, worin die anderen Substanzen Reagenzien zur Markierung umfassen.

14. Verfahren zum Ausführen eines Nukleinsäurehybridisierungs-Assays, umfassend das Behandeln einer markierten Nukleinsäureprobe oder eines Precursors dieser, die bzw. der reversibel in getrockneter Form an einer festen Träger gebunden und durch Zugabe von Wasser oder einer wäßrigen Lösung von diesem ablösbar ist, die Zugabe von Wasser oder einer wäßrigen Lösung, um die Probe oder den Precursor abzulösen, das Inkontaktbringen der abgelösten Probe oder des Precursors mit einer zu untersuchenden Probe, umfassend Nukleinsäure, unter solchen Bedingungen, die die Hybridisierung von homologen oder im wesentlichen homologen Sequenzen ermöglichen, das Behandeln des Precursors, sofern notwendig, in geeigneter Weise, um eine markierte Probe herzustellen, das Entfernen der nicht hybridisierten Probe aus der Mischung und das Bestimmen, ob und wieviel der markierten Probe, die an die Nukleinsäuresonde hybridisiert ist, vorliegt, wobei das Assay in Kontakt mit der Oberfläche des festen Trägers durchgeführt wird.

15. Verfahren nach Anspruch 14, wobei die Probe oder der Precursor dieser durch Zugabe einer Hybridisierungsflüssigkeit abgelöst wird.

16. Verfahren nach Anspruch 14 oder 15, wobei ein reversibel an den Träger gebundener Precursor in geeigneter Weise behandelt wird, um eine markierte Probe herzustellen, wobei eine Polymerase verwendet wird, um dem Precursor markierte Nukleotide hinzuzufügen.

17. Verfahren nach einem der Ansprüche 14 bis 16, wobei das Verfahren so durchgeführt wird, daß genau bestimmte Mengen an Reagenzien nicht zusätzlich benötigt werden.

18. Verfahren nach einem der Ansprüche 14 bis 17, wobei das Verfahren zur Chromosomenanalyse von Zellen angewendet wird.

19. Kit zur Verwendung in Nukleinsäurehybridisierungs-Assays, umfassend eine markierte Nukleinsäureprobe oder einen Precursor dieser, die bzw. der reversibel in getrockneter Form an einen festen Träger gebunden und durch Zugabe von Wasser oder einer wäßrigen Lösung von diesem ablösbar ist, zur Durchführung des Assays in Kontakt mit der Oberfläche des festen Trägers und Bedienungsanleitungen.

20. Kit nach Anspruch 19, darüber hinaus umfassend andere Substanzen, die in getrockneter Form an denselben oder einen weiteren festen Träger gebunden sind.

21. Kit nach Anspruch 19 oder 20, worin die anderen Substanzen Regenzien zur Markierung umfassen.

22. Kit nach einem der Ansprüche 19 bis 21, darüber hinaus umfassend eine oder mehrere der folgenden Substanzen: Hybridisierungsflüssigkeit, Waschlösung, Vulkanisierungslösung und eine Lösung, umfassend Propidiumjodid, die das Verblassen verhindert.

## Revendications

1. Composition destinée à être utilisée dans des essais d'hybridation d'acide nucléique, caractérisée en ce qu'une sonde d'hybridation d'acide nucléique marquée, ou un précurseur de celle-ci, sous forme desséchée liée de manière réversible à un support solide et étant libérable de celui-ci dès l'addition d'eau ou d'une solution aqueuse, et en ce que l'essai d'hybridation est effectué en contact avec la surface du support solide auquel la sonde d'acide nucléique est liée de manière réversible.

2. Composition selon la revendication 1, caractérisée en ce qu'elle comprend un précurseur de sonde sous forme d'une sonde d'acide nucléique non marquée.

3. Composition selon la revendication 2, caractérisée en ce que le marquage du précurseur de sonde est réalisé par l'addition à celui-ci de nucléotides marqués.

4. Composition selon l'une quelconque des revendications 1-3, caractérisée en ce que le support solide est essentiellement plan.

5. Composition selon l'une quelconque des revendications précédentes, caractérisée en ce que le support solide comprend une lame ou une lamelle de microscope.

6. Composition selon l'une quelconque des revendications précédentes, caractérisée en ce que le support solide comprend le verre siliconisé.

7. Composition selon l'une quelconque des revendications précédentes, caractérisée en ce qu'une pluralité de différentes sondes ou de précurseurs de celle-ci sont liés de manière réversible à un support solide unique.

8. Composition selon l'une quelconque des revendications précédentes, caractérisée en ce qu'elle comprend en outre d'autres substances sous forme desséchée liée au même ou autre support solide.

9. Composition selon la revendication 8, caractérisée en ce que lesdites autres substances comprennent une substance immobilisée de manière réversible ou irréversible, capable d'interagir avec la sonde d'acide nucléique.

10. Composition selon la revendication 8 ou 9, caractérisée en ce que lesdites autres substances comprennent une enzyme.

11. Composition selon l'une quelconque des revendications 8, 9 ou 10, caractérisée en ce que lesdites autres substances comprennent une ADN polymérase.

12. Composition selon l'une quelconque des revendications 8-11, caractérisée en ce que lesdites autres substances comprennent des désoxynucléotides en vue d'une addition à la substance sonde.

13. Composition selon l'une quelconque des revendications 8-12, caractérisée en ce que lesdites autres substances comprennent des réactifs de marquage.

14. Procédé de mise en oeuvre d'un essai d'hybridation d'acide nucléique, caractérisé en ce qu'il comprend: le traitement d'une sonde d'acide nucléique marquée, ou d'un précurseur de celle-ci, sous forme desséchée liée de manière réversible à un support solide et étant libérable de celui-ci dès l'addition d'eau ou d'une solution aqueuse par l'addition d'eau ou d'une solution aqueuse pour libérer la sonde ou le précurseur; la mise en contact de la sonde ou du précurseur libérés avec un échantillon à doser, comprenant un acide nucléique, dans des conditions permettant l'hybridation de séquences homologues ou sensiblement homologues; le cas échéant, le traitement du précurseur d'une manière appropriée pour produire une sonde marquée, l'élimination de la sonde non hybridée du mélange et la mise en évidence de la présence (s'il y en a) de la sonde marquée hybridée à l'acide nucléique de l'échantillon, et en ce que le dosage est mis en oeuvre en contact avec la surface du support solide.

15. Procédé selon la revendication 14, caractérisé en ce que la sonde ou le précurseur de celle-ci est libéré par l'addition de liquide d'hybridation.

16. Procédé selon la revendication 14 ou 15, caractérisé en ce qu'un précurseur lié de manière réversible au support est traité d'une manière appropriée pour produire une sonde marquée par l'utilisation d'une polymérase pour ajouter des nucléotides marqués au précurseur.

17. Procédé selon l'une quelconque des revendications 14-16, caractérisé en ce que le procédé est mis en oeuvre d'une manière ne nécessitant pas des quantités supplémentaires de réactifs dosées de manière précise.

18. Procédé selon l'une quelconque des revendications 14-17, caractérisé en ce que le procédé est appliqué à l'analyse chromosomique des cellules.

19. Kit destiné à être utilisé dans des essais d'hybridation d'acide nucléique, caractérisé en ce qu'il comprend: une sonde d'acide nucléique marquée ou un précurseur de celle-ci, sous forme desséchée liée de manière réversible à un support solide et étant libérable de celui-ci dès l'addition d'eau ou d'une solution aqueuse, pour la mise en oeuvre de l'essai en contact avec la surface du support solide, et un mode d'emploi.

20. Kit selon la revendication 19, caractérisé en ce qu'il comprend en outre d'autres substances sous forme desséchée liée au même ou autre support solide.

21. Kit selon la revendication 19 ou 20, caractérisé en ce que lesdites autres substances comprennent des réactifs de marquage.

22. Kit selon l'une quelconque des revendications 19-21, caractérisé en ce qu'il comprend en outre un ou plusieurs de ce qui suit: un liquide d'hybridation; une solution de lavage; une solution de vulcanisation; et une solution anti-décoloration comprenant de l'iodure de propidium.
